Europäisches Patentamt

European Patent Office

Office européen des brevets

(19)

(11) Publication number: **0 406 210 B1**

(12) **EUROPEAN PATENT SPECIFICATION**

(45) Date of publication of patent specification: **27.09.95**

(51) Int. Cl.6: **A61K 31/70, A61K 9/08**

(21) Application number: **90870103.0**

(22) Date of filing: **26.06.90**

(54) **Ophthalmic composition.**

(30) Priority: **29.06.89 US 373725**

(43) Date of publication of application:
**02.01.91 Bulletin 91/01**

(45) Publication of the grant of the patent:
**27.09.95 Bulletin 95/39**

(84) Designated Contracting States:
**AT BE CH DE DK ES FR GB GR IT LI LU NL SE**

(56) References cited:
**EP-A- 0 234 854**
**WO-A-88/05306**

**AM. J. PHYSIOL., vol. 252, no. 3, part 2, 1987, pages 551-559, American Physiological Society; M.L. ZEIDEL et al**

**CURR. EYE RES., vol. 6, no. 10, 1987, pages 1189-1196, IRL Press Ltd, Oxford,GB; T.W. MITTAG et al.: "Atrial natriuretic peptide (ANP), guanylate cyclase,and intraocular pressure in the rabbit eye"**

(73) Proprietor: **WASHINGTON UNIVERSITY**
**Campus Box 1137,**
**1 Brookings Drive**
**St. Louis,**
**Missouri 63130-4899 (US)**

(72) Inventor: **Becker, Bernard**
**8655 West Kingsbury**
**St. Louis,**
**Missouri 63124 (US)**

(74) Representative: **Ernst, Hubert et al**
**Monsanto Services International SA/NV**
**Avenue de Tervuren 270-272**
**Letter Box 21**
**B-1150 Brussels (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

JOURNAL OF PHARMACEUTICAL SCIENCES, vol. 64, no. 1, January 1975, pages 1-37;M.S. AMER et al.: "Cyclic nucleotide phosphodiesterases: properties,activators, inhibitors, structure-activity relationships, and possible role indrug development"

PROC. NATL. ACAD. SCI. USA, vol. 82, no. 24, December 1985, pages 8813-8817; A.L. ZIM-MERMAN et al.: "Interaction of hydrolysis-resistant analogs of cyclic GMPwith the phosphodiesterase and light-sensitive channel of retinal rod outersegments"

Dorland's Illustrated Medical Dictionary, 25th ed., 1974, pages 650, 1543, plate XV

AMA Drug Evaluations, 4th ed., 1980, pages 350, 351, 404-406

**Description**

This invention relates to an ophthalmic composition suitable for topical administration to the eye of a mammal for decreasing intraocular pressure and to the use of defined analogs of cyclic guanosine monophosphate for the manufacture of an ophthalmic composition for decreasing intraocular pressure in a mammal.

Glaucoma is a disorder characterized by increased intraocular pressure (IOP) that may cause impaired vision which, if sufficiently high and persistent, leads to irreversible blindness. Conventional treatment for control of the disease usually begins with the administration of eyedrops containing dilute preparations of drugs such as pilocarpine, timolol maleate and, in some cases, carbachol or cholinesterase inhibitors such as echothiophate iodide or carbonic anhydride inhibitors such as dichlorophenamide or acetazolamide.

In recent years, a major new hormonal system of bioactive peptides has been discovered, known as the atriopeptins or atrial peptides (APs), which are released from cardiac myocytes and may play important roles in fluid and electrolyte homeostasis. See reviews by Needleman et al., Hypertension 7(4), 469-482 (1985); Needleman, N. Engl. J. Med. 314, 828-834 (1986); and Needleman, Fed. Proc. 45(7), 2096-2100 (1986).

AP receptors are often associated with the particulate form of the enzyme guanylate cyclase and are found in high concentrations in rabbit ciliary epithelium [Bianchi et al., Curr. Eye Res. 5 283 (1986)]. When stimulated by AP, ciliary epithelium in vitro produces increased amounts of cyclic guanosine monophosphate (cGMP) [Nathanson, Invest. Ophthalmol. Vis. Sci. 28, 1357 (1987)]. In vivo, 4-48 hours after the injection of AP into the vitreous cavity of the rabbit eye, significant decreases in IOP (5-8 mm Hg) [Nathanson et al., Ibid.; Sugrue and Viader, Eur. J. Pharmacol. 130, 349 (1986); and Korenfeld and Becker, Invest. Ophthalmol. Vis. Sci. 30, In Press (1989)] and increases in anterior chamber agueous humor concentration of cGMP (15-30 fold) are observed (unpublished observations by the present inventor). The decrease in IOP is associated with a decrease in aqueous flow as estimated by fluorophotometry, tonography and changes in agueous humor ascorbate concentrations [Korenfeld and Becker, Ibid.]. The topical application of nitrovasodilators, such as nitroglycerine and sodium nitroprusside, which activate the soluble form of the enzyme guanylate cyclase, also increase aqueous humor cGMP and lover IOP (unpublished observations by the present inventor). See also Nathanson, Eur. J. Pharmacol. 147, 155-156 (1988).

EP-A-0 234 854 discloses pharmaceutical compositions containing phosphodiesterase inhibitors or cyclic nucleotide analogues in admixture with a pharmaceutically acceptable carrier or diluent and the use thereof to stimulate tear secretion. EP-A-0 234 854 is silent about a possible effect of the disclosed compounds and compositions on intraocular pressure.

WO-A-8 805 306 discloses the treatment of cranial volume dysfunctions, including fluid volume dysfunctions in the eye, with atriopeptins and nitrogen-containing guanylate cyclase activators that are inhibitors of phosphodiesterases, and a combination thereof, which effect an increase in cyclic guanosine monophosphate (cGMP) at the site of the dysfunction or at the site of the synthesis or removal of the accumulating fluid, resulting, inter alia, in intraocular pressure (IOP) reduction.

A.L. Zimmerman et al., Proc. Natl. Acad. Sci. USA, Vol. 82, 8813-8817, (1985), disclose the use of 8-bromo cyclic guanosine monophosphate to open cation-selective channels when applied to the cytoplasmic side of excised patches of membrane from retinal rod outer segments (ROS). A.L. Zimmerman et al. do not make any disclosure about the possible use of 8-bromo-cGMP or analogs thereof for reducing intraocular pressure.

M.S. Amer and W.E. Kreighbaum in Journal of Pharmaceutical Sciences, Vol. 64, 1-37, (1975), a review article, describe cyclic nucleotide derivatives, e.g. cGMP, and their structure-activity relationship with respect to phosphodiesterase inhibition, but do not disclose any relation with respect to intraocular pressure reduction.

Brief Description of the Invention

In accordance with the present invention, a novel ophthalmic composition suitable for topical administration to the eye of a mammal for decreasing intraocular pressure (IOP) and the use of defined analogs of cyclic guanosine monophosphate and their alkali metal salts for the manufacture of such ophthalmic composition are provided. The compositions for administering topically to the eye of a mammal are containing an effective amount for decreasing IOP of an analog of cyclic GMP which is more resistant to hydrolysis by phosphodiesterases than cyclic GMP. In particular, these analogs of cyclic GMP are the 8-bromo-, and/or $N^2$-butyryl- and/or 2'-O-butyryl-substituted cyclic GMPs.

These useful analogs of cyclic GMP for decreasing IOP are illustrated by the following specific compounds and their alkali metal salts, e.g. sodium salts:

8-Bromoguanosine 3',5'-cyclic
monophosphate,
$N^2$,2'-O-Dibutyrylguanosine 3'-5'-
cyclic monophosphate, 2'-O-Monobutyrylguanosine 3',5'-cyclic
monophosphate,
$N^2$-Monobutyrylguanosine 3',5'-
cyclic monophosphate,
$N^2$-Monobutyryl-8-bromoguanosine
3',5'-cyclic monophosphate, and
2'-O-Monobutyryl-8-bromoguanosine
3',5'-cyclic monophosphate.

Although 8-bromoguanosine 3',5'-cyclic monophosphate (8-BrcGMP) is known to mimic the effect of AP in several secretory sites [O'Grady et al., Amer. J. Physiol. (Cell Physiol. 18), C531-C534 (1985); Zeidel et al., Amer. J. Physiol. (Renal Fluid Electrolyte Physiol. 21), F551-F559 (1987)], and has been proposed as a stimulant for tear secretion [U.S. Patent 4,753,945], it has not been known heretofore as useful for decreasing IOP in ophthalmic compositions as disclosed herein.

Detailed Description of the Invention

While the specification concludes with claims particularly pointing out and distinctly claiming the subject matter regarded as forming the present invention, it is believed that the invention will be better understood from the following detailed description of preferred embodiments of the invention taken in conjunction with the accompanying drawings in which:

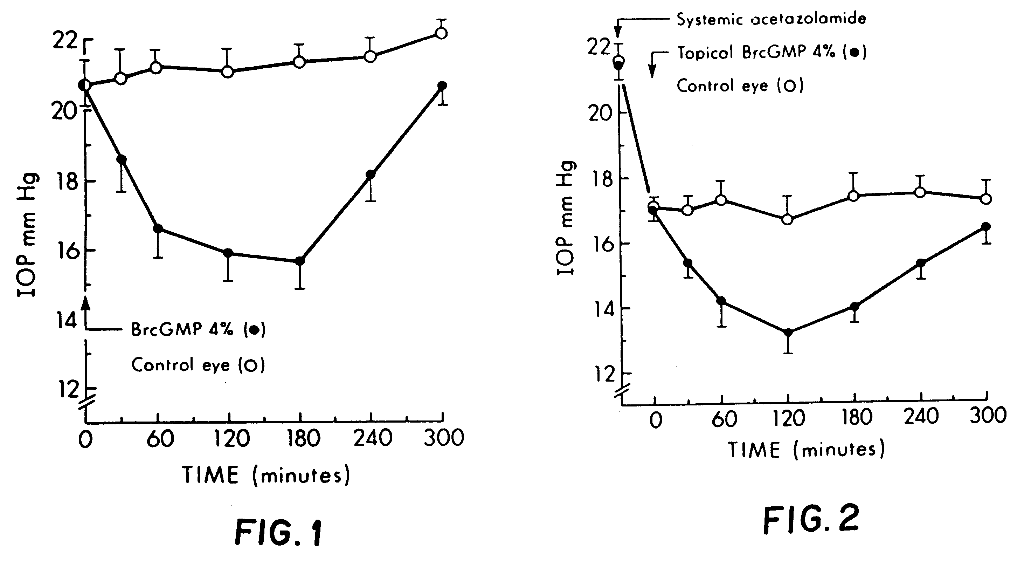

FIG. 1 is a graphical representation which shows the effect of topical 8-BrcGMP 4% (50 $\mu$l x 2) on IOP (mean ± SEM for 10 rabbits). Differences in IOP between treated experimental and contralateral control eyes (corrected for differences at time 0) are significant ($p < 0.001$) at times 30 to 240 minutes.

FIG. 2 is a graphical representation which shows the effect of topical 8-BrcGMP 4% (50 $\mu$l x 2) on IOP (mean ± SEM) of 10 rabbits treated with systemic acetazolamide (50 mg/kg iv + 50 mg/kg sc) administered 30 minutes before topical 8-BrcGMP 4%. Decreases in IOP in both eyes 30 minutes following acetazolamide are significant ($p < 0.001$). Differences in IOP between 8-BrcGMP treated experimental and contralateral control eyes are significant after 30 minutes ($p < 0.01$) and at times 60 to 240 minutes ($p < 0.001$).

The effective amount of the cGMP analog in the ophthalmic composition generally ranges from 0.05% to 10% by volume, with maximum effects at the upper part of the range. Illustratively, the topical application of 8-BrcGMP produced significant decreases in IOP in rabbit eyes. Maximum effects were obtained with a 4% concentration and IOP was reduced significantly ($p < 0.001$) between 30 and 240 minutes after administration of the agent. The fall in IOP occurred without significant change in tonographic outflow facility. A significant further decrease in IOP was induced by topical 8-BrcGMP in rabbits whose IOP had been lowered by the systemic administration of the carbonic anhydrase inhibitor, acetazolamide.

The active cGMP analog drug is preferably formulated in agueous solution and administered as eyedrops by introduction into the cul-de-sac of the eye. It can also be administered as an ointment or in a controlled release system such as the Ocusert * system as described, e.g., in U.S. Patents 3,618,604 and 3,828,777. *(Registered Trademark).

The active cGMP analog drug is incorporated in the ophthalmic composition of the invention in admixture with a diluent or carrier medium which is non-toxic to the eye and surrounding tissues or otherwise pharmaceutically acceptable and which is compatible with the drug. Representative diluents or carriers are water, saline, glycerin, propylene glycol, emulsifying and suspending agents such as methylcellulose mixed with water, mixtures of propylene glycol monostearate and oils, sodium alginate, gum tragacanth, polyoxyethylene monostearate, polyvinylpyrrolidone, polyvinyl alcohol and the like materials. Minor amounts of salts, acids and buffering agents such as sodium chloride, potassium chloride, sodium phosphate (monobasic and debasic), boric acid, ethylene diamine tetraacetate and preservatives, e.g. benzalkonium chloride, can also be included in the ophthalmic composition. Physiologic saline (0.9% NaCl) is a preferred medium for the active cGMP analog drug.

The following examples will illustrate the invention in greater detail although it will be appreciated that the invention is not limited to these specific examples.

4

Materials and Methods

Adult New Zealand albino rabbits (2-4 kg) of both sexes were housed and handled in accordance with the ARVO Resolution for Animal Care. Animals were exposed to 12 hour light/dark cycles in a constant temperature isolated environment for at least two weeks before use. They had free access to water and food and were handled repeatedly by only one trained technician. Intraocular pressures were measured on unrestrained rabbits with a manometrically calibrated Pneumotonometer using topical 0.5% proparacaine. Preliminary IOP measurements were made to accustom the animals to the procedure. Tonography was carried out with a hand-held Schiotz tonometer using the 5.5 gram weight and readings every 15 seconds for 4 minutes. Outflow facility values (C) were estimated from tables calculated for human eyes.

8-BrcGMP was purchased from Sigma Chemical Company, St. Louis, Missouri. For topical use fresh solutions were made daily in sterile 0.9% NaCl solution. Fifty $\mu$l drops were administered to the experimental eye at time 0 and repeated at 5 minutes. Vehicle was used in contralateral control eyes. IOP was measured at times 0, 30, 60, 120, 180, 240, and 300 minutes after topical administration. Sodium acetazolamide was purchased from American Cyanamid Company, Pearl River, NY, and injected in doses of 50 mg/kg intravenously and 50 mg/kg subcutaneously. A two-tailed paired t-test was utilized to compare experimental and control eyes (each corrected for its baseline value).

Results

EXAMPLE 1

Intraocular pressure and tonography

Following the topical administration of 8-BrcGMP 4%, IOP was decreased significantly ($p < 0.001$) at all time intervals between 30 and 240 minutes in the experimental eye without significant effect on the contralateral control eye (Figure 1). Maximum effect (approximately 23-25% fall in IOP) occurred between 60 and 180 minutes with recovery to baseline levels by 5-6 hours. Dose response studies for the same time intervals after applications of 0.5% to 8.0% concentrations of 8-BrcGMP revealed that maximum pressure lowering was accomplished with 4.0% concentration; higher concentrations produced no greater effect on IOP but tended to prolong the effect beyond 4 hours (Table 1). Tonographic data 2 hours after repeated doses of topical 8-BrcGMP 2% demonstrated significant decreases in IOP without change in outflow facility (Table 2).

Table 1

IOP Dose Response to Topical 8 BrcGMP (mm HG)

Time (mins)

| Concn 8 BrcGMP | 30 | 60 | 120 | 180 | 240 | 300 |
|---|---|---|---|---|---|---|
| 0.5% | 0 ± 0.5 | -0.2 ± 0.3 | -0.9 ± 0.7 | -0.7 ± 0.2 | -0.5 ± 0.2 | -0.4 ± 0.2 |
| 1.0% | -0.9 ± 0.7 | -1.1 ± 0.5*** | -2.5 ± 0.5** | -2.3 ± 0.6** | -0.8 ± 0.4 | -0.1 ± 0.3 |
| 2.0% | -2.1 ± 0.6 ** | -4.1 ± 0.9* | -4.1 ± 0.6* | -4.3 ± 0.7* | -2.7 ± 0.7** | -1.1 ± 0.6 |
| 4.0% | -2.3 ± 0.4* | -4.6 ± 0.7* | -5.1 ± 0.7* | -5.6 ± 0.9* | -3.3 ± 0.5* | -1.5 ± 0.6*** |
| 8.0% | -1.8 ± 0.3* | -3.9 ± 0.5* | -5.3 ± 0.6* | -5.3 ± 0.7* | -4.8 ± 0.8* | -3.3 ± 0.9** |

Values are differences in IOP (mean ± SEM) between experimental and contralateral

control eyes (corrected for differences at time 0) at various times after topical

administration of 8BrcGMP.  N = 10 rabbits at each concentration.

*       p < 0.001

**      p < 0.01

***     p < 0.05

EP 0 406 210 B1

EP 0 406 210 B1

Table 2

| Tonography 2 Hours After Topical 8 BrcGMP 2% | | |
|---|---|---|
| | Intraocular pressure (mm Hg) | Outflow facility ($\mu$l/min/mm Hg) |
| Control eye | 19.7 ± 0.8 | 0.37 ± 0.02 |
| Experimental eye | 15.1 ± 0.6* | 0.34 ± 0.08 |
| Topical 8 BrcGMP 2% (50 $\mu$l) to experimental eye at 0, 30 and 60 minutes. Values are mean ± SEM for 7 rabbits. | | |

\* p > 0.001 for difference between control and experimental eye.

EXAMPLE 2

Acetazolamide

The administration of acetazolamide to 10 rabbits 30 minutes before the topical application of 8-BrcGMP 4% as in Example 1, above, produced a significant ($p < 0.001$) and almost identical fall in IOP of 4.5 ± 0.7 mm Hg in both eyes at time 0 (Figure 2). The subsequent application of 8-BrcGMP 4% to one eye and diluent to the contralateral control eye resulted in a significant ($p < 0.001$) further decrease in IOP in the treated eye without change in the control eye. The decrease was slightly less in absolute magnitude but similar in relative change (approximately 20 to 23%) when compared with animals not pretreated with acetazolamide.

Similar effective decrease of IOP is obtained as in Examples 1 and 2, above, when the $N^2$-butyryl-, 2'-O-butyryl, $N^2$,2'-O-dibutyryl, $N^2$-butyryl-8-bromo-, and 2'-O-butyryl-8-bromo-substituted guanosine cyclic monophosphates are used instead of the 8-bromo-substituted cGMP in said Examples.

**Claims**

1. An ophthalmic composition suitable for topical administration to the eye of a mammal for decreasing intraocular pressure, said composition comprising an analog of cyclic guanosine monophosphate (GMP) selected from :

    $N^2$,2'-O-Dibutyrylguanosine 3',5'-cyclic monophosphate,

    2'-O-Monobutyrylguanosine 3',5'-cyclic monophosphate,

    $N^2$-Monobutyrylguanosine 3',5'-cyclic monophosphate,

    $N^2$-Monobutyryl-8-bromoguanosine 3',5'-cyclic monophosphate,

    2'-O-Monobutyryl-8-bromoguanosine 3',5'-cyclic monophosphate,

    and their alkali metal salts,

    in admixture with a pharmaceutically acceptable carrier or diluent.

2. The composition of claim 1 in which the amount of the active analog of cyclic GMP is 0.05% to 10% by volume of the composition.

3. The composition of claim 1 in which the amount of active analog of cyclic GMP is about 4% by volume of the composition.

4. Use of an analog of cyclic guanosine monophosphate (GMP) selected from :

    8-Bromoguanosine 3',5'-cyclic monophosphate,

    $N^2$,2'-O-Dibutyrylguanosine 3',5'-cyclic monophosphate,

    2'-O-Monobutyrylguanosine 3',5'-cyclic monophosphate,

    $N^2$-Monobutyrylguanosine 3',5'-cyclic monophosphate,

    $N^2$-Monobutyryl-8-bromoguanosine 3',5'-cyclic monophosphate,

    2'-O-Monobutyryl-8-bromoguanosine 3',5'-cyclic monophosphate,

    and their alkali metal salts,

    for the manufacture of an ophthalmic composition for decreasing intraocular pressure in a mammal.

7

**Patentansprüche**

1. Ophthalmische Zusammensetzung, die zur topischen Verabreichung an das Auge eines Säugers für eine Senkung des Augendrucks geeignet ist, welche Zusammensetzung umfaßt: ein Analogon von cyclischem Guanosinmonophosphat (GMP), ausgewählt aus:
   cyclischem $N^2,2'$-O-Dibutyrylguanosin-3',5'-monophosphat,
   cyclischem 2'-O-Monobutyrylguanosin-3',5'-monophosphat,
   cyclischem $N^2$-Monobutyrylguanosin-3',5'-monophosphat,
   cyclischem $N^2$-Monobutyryl-8-bromguanosin-3',5'-monophosphat,
   cyclischem 2'-O-Monobutyryl-8-bromguanosin-3',5'-monophosphat,
   und ihren Alkalimetallsalzen,
   gemischt mit einem pharmazeutisch annehmbaren Träger oder Verdünnungsmittel.

2. Zusammensetzung nach Anspruch 1, worin die Menge des aktiven Analogons von cyclischem GMP 0,05 Vol.% bis 10 Vol.% der Zusammensetzung beträgt.

3. Zusammensetzung nach Anspruch 1, worin die Menge des aktiven Analogons von cyclischem GMP etwa 4 Vol.% der Zusammensetzung beträgt.

4. Verwendung eines Analogons von cyclischem Guanosinmonophosphat (GMP), ausgewählt aus:
   cyclischem 8-Bromguanosin-3',5'-monophosphat,
   cyclischem $N^2,2'$-O-Dibutyrylguanosin-3',5'-monophosphat,
   cyclischem 2'-O-Monobutyrylguanosin-3',5'-monophosphat,
   cyclischem $N^2$-Monobutyrylguanosin-3',5'-monophosphat,
   cyclischem $N^2$-Monobutyryl-8-bromguanosin-3',5'-monophosphat,
   cyclischem 2'-O-Monobutyryl-8-bromguanosin-3',5'-monophosphat,
   und ihren Alkalimetallsalzen,
   zur Herstellung einer ophthalmischen Zusammensetzung zur Senkung des Augendrucks bei einem Säuger.

**Revendications**

1. Composition ophtalmique appropriée pour l'administration topique dans l'oeil de mammifère destinée à réduire la pression intraoculaire, ladite composition contenant un analogue de guanosine-monophosphate cyclique (GMP) choisi parmi
   le $N^2,2'$-O-dibutyrylguanosine-monophosphate 3',5'-cyclique
   le 2'-O-monobutyrylguanosine-monophosphate 3,5'-cylique
   le $N^2$-monobutyryl-guanosine-monophosphate 3',5'-cyclique
   le $N^2$-monobutyryl-8-bromoguanosine-monophosphate 3',5'-cyclique
   le 2'-O-monobutyryl-8-bromoguanosine-monophosphate 3,5'-cylique ainsi que leur sels de métal alcalin,
   sous forme de mélange avec un excipient ou diluant pharmaceutiquement acceptable.

2. Composition conforme à la revendication 1 dans laquelle la quantité d'analogue actif de GMP cyclique est comprise entre 0,05 % et 10 % en volume de la composition.

3. Composition conforme à la revendication 1 dans laquelle la quantité d'analogue actif de GMP cyclique est d'environ 4 % en volume de la composition.

4. Utilisation d'un guanosine-monophosphate cyclique (GMP) choisi parmi
   le 8-bromoguanosine-monophosphate 3',5' cyclique
   le $N^2,2'$-O-dibutyrylguanosine-monophosphate 3',5' cyclique
   le 2'-O-monobutyrylguanosine-monophosphate 3,5' cylique
   le $N^2$-monobutyryl-guanosine-monophosphate 3',5' cyclique
   le $N^2$-monobutyryl-8-bromoguanosine-monophosphate 3',5' cyclique
   le 2'-O-monobutyryl-8-bromoguanosine-monophosphate 3,5' cylique
   ainsi que leur sels de métal alcalin,
   pour la fabrication d'une composition opthalmique destinée à réduire la pression intraoculaire chez les

mammifères.

**FIG. 1**

**FIG. 2**

EP 0 406 210 B1